(19) Europäisches Patentamt
European Patent Office
Office européen des brevets

(11) Veröffentlichungsnummer: **0 097 617**
**B1**

# (12) EUROPÄISCHE PATENTSCHRIFT

(45) Veröffentlichungstag der Patentschrift: **29.01.86**

(51) Int. Cl.⁴: **C 07 D 249/08**, C 07 C 109/04, C 07 C 109/12

(21) Anmeldenummer: **83810259.8**

(22) Anmeldetag: **13.06.83**

(54) **Verfahren zur Herstellung von 1-(2-(2,4-Dichlorphenyl)pentyl)-1H-1,2,4-triazol und neue Hydrazon- oder Hydrazinderivate.**

(30) Priorität: **18.06.82 CH 3773/82**

(43) Veröffentlichungstag der Anmeldung:
**04.01.84 Patentblatt 84/1**

(45) Bekanntmachung des Hinweises auf die Patenterteilung:
**29.01.86 Patentblatt 86/5**

(84) Benannte Vertragsstaaten:
**AT BE CH DE FR GB IT LI NL SE**

(56) Entgegenhaltungen:
**DE - A - 2 735 872**
**DE - B - 1 098 950**
**DE - B - 1 213 834**

**CHEMICAL ABSTRACTS, vol. 97, no. 3, 19. Juli 1982, Columbus, Ohio, USA SCHARER MAX et al. "Azole microbicide" Seite 287, Spalte 2; Zusammenfassung-Nr. 19 058z**
**CHEMICAL ABSTRACTS, vol. 97, no. 17, 25. Oktober 1982, Columbus, Ohio, USA VAN GESTEL et al. "Composition for protecting wood and coating against microbial degradation, methods for protecting wood, as well as wood coatings and an antiseptic detergent composition" Seite 257, Spalte 2; Zusammenfassung-Nr. 140 274v**

(73) Patentinhaber: **CIBA-GEIGY AG, Klybeckstrasse 141, CH-4002 Basel (CH)**

(72) Erfinder: **Wetter, Hansjürg, Dr., Vogesenstrasse 22, CH-4106 Therwil (CH)**
Erfinder: **Baumeister, Peter, St. Annaweg 24, CH-4113 Flüh (CH)**
Erfinder: **Radimerski, Paul, Dr., Hauptstrasse 30, CH-4411 Arisdorf (CH)**
Erfinder: **Martin, Pierre, Dr., Meisenweg 38, CH-4310 Rheinfelden (CH)**

**EP 0 097 617 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die vorliegende Erfindung betrifft ein neues Verfahren zur Herstellung von 1-[2-(2,4-Dichlorphenyl)pentyl]-1H-1,2,4-triazol sowie für dessen Herstellung entwickelte neue Hydrazon- oder Hydrazinderivate.

Aus der DE-OS 2735872 ist bekannt, dass man fungizide 1-(2-Aryläthyl)-1H-1,2,4-triazole, u.a. das 1-[2-(2,4-Dichlorphenyl)pentyl]-1H-1,2,4-triazol, durch Umsetzung von 1H-1,2,4-Triazol oder einem Alkalimetallsalz davon mit einem reaktiven Ester geeigneter 2-Aryläthylderivate $X-CH_2CH$ (Ar)(R) (Ar=gegebenenfalls substituiertes Aryl, R= u.a. Alkyl, X=z.B. $-OSO_2CH_3$) erhalten kann. Dabei entstehen Isomerengemische von 1H-1,2,4- und 1H-1,3,4-Triazolderivaten, wovon nur die ersteren fungizide Wirkung zeigen. Diese Isomerengemische lassen sich nur mit sehr grossem Aufwand trennen. Die genannten reaktiven Ester müssen ihrerseits durch eine mehrstufige Synthese hergestellt werden, indem man entsprechende Arylacetonitrile NC−CH(Ar)(R) in Gegenwart einer starken nicht oxidierenden Säure, wie HCl, in Ester R'OOC-CH(Ar)(R) überführt. Diese werden anschliessend in Gegenwart eines Alkalimetallhydrids, wie Lithiumaluminiumhydrid oder Lithiumborhydrid, zu Alkoholen $HOCH_2$-CH(Ar)(R) reduziert. Schliesslich werden diese Alkohole durch Behandeln mit z.B. Methansulfonylchlorid in die genannten reaktiven Ester übergeführt. Bei diesem vorbekannten Verfahren entstehen wie schon erwähnt beträchtliche Anteile an unwirksamen 1H-1,3,4-Triazolderivaten.

Zur Herstellung der Alkohole müssen relativ hohe Mengen der für grosstechnische Anwendungen nur beschränkt verfügbaren Alkalimetallhydride eingesetzt werden. Die Verwendung derartiger Hydride ist zudem aus sicherheitstechnischen Gründen (Feuer- und Explosionsgefahr) unerwünscht. Katalytisch lassen sich die Ester R'OOC−CH(Ar)(R) nicht oder nur in sehr unbefriedigenden Ausbeuten reduzieren. Aus den genannten Gründen hat dieses vorbekannte Verfahren keine technische Bedeutung erlangt.

Aufgabe der Erfindung war daher die Bereitstellung eines neuen, auch grosstechnisch anwendbaren Verfahrens, mit dem sich die obigen Nachteile vermeiden lassen und mit dem 1-[2-(2,4-Dichlorphenyl)pentyl]-1H-1,2,4-triazol in guten bis sehr guten Ausbeuten und vor allem in isomerenfreier Form hergestellt werden kann.

Nach dem neuen erfindungsgemässen Verfahren kann das 1-[2-(2,4-Dichlorphenyl)pentyl]-1H-1,2,4-triazol der Formel I

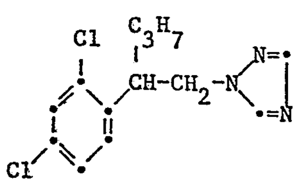 (I)

auf einfache und wirtschaftliche Weise in isomerenfreier Form dadurch hergestellt werden, dass man 2-(2,4-Dichlorphenyl)valeronitril in Gegenwart von Wasserstoff, einer Säure und eines Hydrierungskatalysators mit einer Verbindung der Formel II

$$H_2N-NH-R \qquad (II)$$

zu einer Verbindung der Formel III

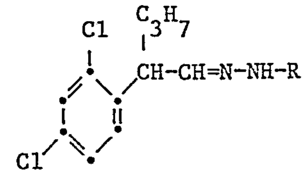 (III)

umsetzt, die Verbindung der Formel III katalytisch zu einer Verbindung der Formel IV

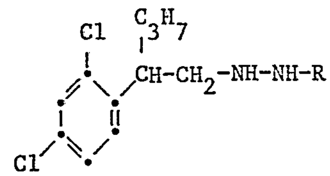 (IV)

hydriert, anschliessend entweder a) Verbindungen der Formel IV mit R ungleich Wasserstoff hydrolysiert und die Verbindung der Formel IV mit R=H oder Salze davon mit anorganischen oder organischen Säuren mit Formamid und/oder [3-(Dimethylamino)-2-azaprop-2-en-1-yliden]-dimethylammoniumchlorid (Azasalz); $[(CH_3)_2 N^+\!=CH-N=CH-N(CH_3)_2]Cl^-$) in die Verbindung der Formel I überführt, oder b) Verbindungen der Formel IV mit R=−COR' mit wässeriger Ameisensäure in die N,N'-Bisformylderivate überführt und diese mit Formamid, gegebenenfalls in Gegenwart von $NH_3$ oder einer $NH_3$ liefernden Substanz zur Verbindung der Formel I umsetzt, wobei R Wasserstoff, −CHO, −COR', −COOR' oder $-CONH_2$ und R' $C_{1-4}$-Alkyl, Benzyl oder Phenyl bedeuten.

Alkylgruppen R' können geradkettig oder verzweigt sein, sind aber bevorzugt geradkettig. Als Beispiele seien genannt: Methyl, Äthyl, n-Propyl, Isopropyl, n-Butyl, sek.-Butyl und tert.-Butyl. R stellt vorzugsweise −CHO, −COR' oder −COOR' dar, wobei R' Methyl oder Äthyl ist. Besonders bevorzugt ist R eine Gruppe −COR", wobei R" Wasserstoff, Methyl oder Äthyl bedeutet.

Die Ausgangsprodukte der Formel II und das 2-(2,4-Dichlorphenyl)valeronitril sind bekannt oder können nach an sich bekannten Methoden hergestellt werden, das letztere z.B. durch Alkylierung von 2,4-Dichlorphenylacetonitril nach üblichen Methoden.

Die Umsetzung des 2-(2,4-Dichlorphenyl)valeronitrils mit dem Hydrazin der Formel II wird zweckmässig in organischem oder wässerig-organischem Medium durchgeführt. Geeignete organische Lösungsmittel sind beispielsweise Alkanole mit bis zu 6 C-Atomen, wie Methanol, Äthanol, n-Propanol, Isopropanol, Butanole und Pentanole; aliphatische und cyclische Äther, wie Diäthyläther, Di-n-propyläther, Diisopropyläther, Tetrahydropyran, Tetrahydrofuran und Dioxan; aromatische und aliphatische Kohlenwasserstoffe, wie Benzol, Toluol, Xylole, n-Hexan und n-Heptan; cyclische oder aliphatische Amide, wie N-Methyl-2-pyrrolidon- und N-Acetyl-2-pyrrolidon; Formamid und N,N-Dialkylamide von aliphatischen Monocarbonsäuren mit 1-3 C-Atomen im Säureteil, wie N,N-Dimethylformamid, N,N-Dimethylacetamid und N,N-Diäthylacetamid, und/oder Gemische der genannten Lösungsmittel mit Wasser. Bevorzugt wird die Umsetzung in einem $C_{1-4}$-Alkanol, besonders Methanol oder Äthanol, oder Gemischen davon mit Wasser vorgenommen. Die Reaktionstemperaturen für diese Umsetzung sind nicht kritisch; bevorzugt sind Reaktionstemperaturen zwischen 0 und 150° C, insbesondere 20 und 60° C. Die Reaktion kann bei Normaldruck oder unter Druck, vorzugsweise zwischen Normaldruck und einem Druck von bis zu 10 bar, vor allem bis zu 4 bar, durchgeführt werden.

Als Hydrierungskatalysatoren können an sich bekannte Katalysatortypen eingesetzt werden. Besonders geeignet sind Kobalt-, Nickel- und Edelmetallkatalysatoren, wie Platin-, Rhodium-, Palladium- und Ruthenium-Katalysatoren. Besonders bevorzugt verwendet man Raney-Nickel oder Rhodium auf Trägern, wie Aktivkohle oder Aluminiumoxid.

Als Säuren eignen sich sowohl anorganische als auch organische Säuren, besonders anorganische und organische Protonensäuren. Beispiele anorganischer Protonensäuren sind Halogenwasserstoffsäuren, wie HCl, HBr und HF, Phosphorsäure und Schwefelsäure. Als organische Protonensäuren kommen z.B. in Betracht: Sulfinsäuren, wie Benzolsulfinsäure; aliphatische und gegebenenfalls substituierte aromatische Sulfonsäuren, wie Methansulfonsäure, Benzolsulfonsäure, p-Toluolsulfonsäure, Naphthalinsulfonsäure, Naphthalindisulfonsäuren; aliphatische Monocarbonsäuren mit vorzugsweise 1-18 C-Atomen, wie Ameisensäure, Essigsäure, Propionsäure, Buttersäure, Laurinsäure, Palmitinsäure, Stearinsäure; halogenhaltige aliphatische Monocarbonsäuren, wie Chloressigsäure, Dichloressigsäure. Trichloressigsäure und Trifluoressigsäure; aliphatische Dicarbonsäuren mit vorzugsweise 2-12 C-Atomen, wie Oxalsäure, Malonsäure, Bernsteinsäure, Adipinsäure und Sebacinsäure; gegebenenfalls substituierte aromatische Mono- und Dicarbonsäuren, wie Benzoesäure, Toluylsäure, Naphthoesäure, Phthalsäure und Terephthalsäure. Bevorzugt sind schwache Säuren, wie aliphatische Monocarbonsäuren mit 1-4 C-Atomen, wie Ameisensäure, Essigsäure, Propionsäure und Buttersäure. Besonders bevorzugt ist Essigsäure.

Das Hydrazin der Formel II und die Säure werden zweckmässig in mindestens äquimolekularer Menge eingesetzt, das Hydrazin bevorzugt in äquimolekularer bis zweifach äquimolekularer Menge und die Säure in äquimolekularer bis vierfach äquimolekularer Menge. Überschüssige Säure kann unter Umständen auch als Lösungsmittel dienen.

Die Umsetzung wird vorzugsweise in Gegenwart von molekularem Wasserstoff durchgeführt. Anstelle von molekularem Wasserstoff können aber auch unter den Reaktionsbedingungen Wasserstoff liefernde Substanzen, wie überschüssiges Hydrazin, unterphosphorige Säure oder deren Salze, verwendet werden.

Die erfindungsgemässe Umsetzung des 2-(2,4-Dichlorphenyl)valeronitrils mit den Hydrazinen der Formel II liefert Hydrazone mit leicht abspaltbaren Schutzgruppen. Dies ist überraschend, da bisher die Überführung von Nitrilgruppen in Hydrazone mit leicht abspaltbaren Gruppen nicht gelang. So wird z.B. in den Chem. Ber., *88*, 1956 (1955) erwähnt, dass diese Methode keine brauchbaren Ergebnisse liefert.

Die Hydrierung der Hydrazone der Formel III zu den Hydrazinen der Formel IV wird mit Vorteil in Gegenwart eines organischen Lösungsmittels vorgenommen. Als solche eignen sich z.B. Alkanole, Äther, Amide und aromatische oder aliphatische Kohlenwasserstoffe der vorerwähnten Art sowie aliphatische Monocarbonsäuren mit 1-5 C-Atomen und Alkylester von aliphatischen Monocarbonsäuren mit insgesamt 2-6 C-Atomen, wie Ameisensäure, Essigsäure, Propionsäure, Buttersäure, Isobuttersäure, Iso- und n-Valeriansäure; Ameisensäuremethyl- und -äthylester, Essigsäuremethyl-, -äthyl-, -n-butyl- und -isobutylester; Ester der Kohlensäure, wie Dimethyl- und Diäthylcarbonat, und Gemische der genannten Lösungsmittel. Besonders bevorzugt sind Essigsäure, Alkanole, wie Methanol, Äthanol, Isopropanol, sek.-Butanol und tert.-Butanol und Gemische dieser Alkohole mit Essigsäureäthylester. Ebenso können Gemische von nicht oxidierenden Mineralsäuren mit den genannten Lösungsmitteln eingesetzt werden.

Als Katalysatoren für die Hydrierung können solche der oben genannten Art verwendet werden, besonders Nickel-, Rhodium-, Ruthenium- und Platin-Katalysatoren, ganz besonders Raney-Nickel oder Rhodium- und Platinkatalysatoren, gegebenenfalls auf Trägern, wie Aktivkohle oder Aluminiumoxid appliziert. Zweckmässig wird bei einem Wasserstoffdruck von 1 bis 200, vor allem 4-100 bar, und bei Temperaturen zwischen 20 und 120° C, besonders 40 und 100° C, gearbeitet.

Hydrazine der Formel IV, worin R ungleich Wasserstoff ist, werden vor dem Ringschluss in an sich bekannter Weise in Gegenwart von Säuren oder Basen zu Verbindungen der Formel IV mit R=H oder Salzen davon hydrolysiert. Als Basen können z.B. Alkalimetall- oder Erdalkalimetallhydroxide oder -carbonate verwendet werden, wie Natrium-, Kalium- und Calciumhydroxid oder -carbonat. Vorzugsweise wird die Hydrolyse in Gegenwart einer starken Säure, besonders einer anorganischen Säure, wie HCl, Schwefelsäure oder Phosphorsäure, durchgeführt. Die Hydrolyse kann in wässerigem oder wässerig-organischem Medium, wie Wasser/Alkanol-Gemischen, besonders Gemischen aus Wasser und Methanol oder Äthanol, erfolgen.

Für den Ringschluss gemäss Verfahrensvariante a) werden das Formamid und/oder das [3-(Dimethylamino)-2-azaprop-2-en-1-yliden]-dimethyl-ammoniumchlorid zweckmässig in mindestens äquimolekularer Menge, bezogen auf die Verbindung der Formel IV (R=H) verwendet. Dabei wird die Umsetzung mit Vorteil in Gegenwart eines inerten organischen Lösungsmittels vorgenommen, z.B. Alkanolen, Estern, Äthern oder Amiden der vorerwähnten Art, Alkylnitrilen mit 2-5 C-Atomen, wie Acetonitril, Propionitril, Butyronitril; Benzonitril; 3-Alkoxypropionitrilen mit 1 oder 2 C-Atomen im Alkoxyteil, wie 3-Methoxypropionitril und 3-Äthoxypropionitril. Bevorzugte Lösungsmittel für die Umsetzung mit dem Azasalz sind $C_{1-5}$-Alkanole, besonders Äthanol. Bei der Umsetzung mit Formamid wird vorzugsweise überschüssiges Formamid als Lösungsmittel eingesetzt. Die Reaktionstemperaturen für den Ringschluss gemäss a) liegen im allgemeinen zwischen 20 und 200° C, bevorzugt zwischen 20 und 180° C.

Für die Formylierung von Verbindungen der Formel IV mit R=–COR', z.B. $-COCH_3$, $-COC_2H_5$ oder $COC_3H_7$, gemäss Verfahrensvariante b) wird zweckmässig 85%ige wässerige Ameisensäure eingesetzt. Die Reaktionstemperaturen liegen dabei bevorzugt zwischen 70 und 100° C. Für den Ringschluss der N,N'-Bisformylderivate wird das Formamid zweckmässig in mindestens äquimolekularer Menge, bezogen auf die Verbindung der Formel IV (R=–COR') verwendet. Als $NH_3$ liefernde Substanzen kommen vor allem Salze von Ammoniak mit schwachen Säuren, z.B. Carbonsäuren, in Betracht. Bevorzugte Salze sind Ammoniumcarbonat, -bicarbonat oder -formiat. Die Reaktionstemperaturen für den Ringschluss der N,N'-Bisformylderivate liegen im allgemeinen zwischen 50 und 200° C, bevorzugt zwischen 120 und 180° C.

Das 1-[2-(2,4-Dichlorphenyl)pentyl]-1H-1,2,4-triazol der Formel I kann gemäss vorliegender Erfindung in besonders vorteilhafter Weise hergestellt werden, wenn man 2-(2,4-Dichlorphenyl)-valeronitril in Gegenwart von Essigsäure und eines Hydrierungskatalysators mit einem Acylhydrazin der Formel IIa

$$H_2N-NH-COR'' \qquad (IIa)$$

zu einem 1-[2-(2,4-Dichlorphenyl)pentyliden]-2-acylhydrazin der Formel IIIa

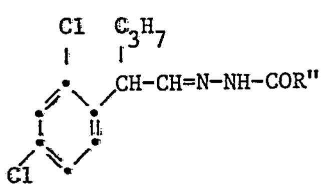

(IIIa)

umsetzt, dieses zum 1-[2-(2,4-Dichlorphenyl)-pentyl]-2-acylhydrazin der Formel IVa

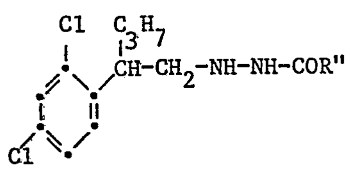

(IVa)

hydriert und dieses durch Umsetzung mit Ameisensäure zunächst in 1-[2-(2,4-Dichlorphenyl)-pentyl]-1,2-diformylhydrazin und dieses durch weitere Umsetzung mit Formamid bei 120-180° C in 1-[2-(2,4-Dichlorphenyl)pentyl]-1H-1,2,4-triazol überführt, wobei R" in Formeln IIa, IIIa und IVa Wasserstoff, Methyl oder Äthyl bedeutet.

Die Hydrazone der Formel III und die Hydrazine der Formel IV bzw. deren Salze können auf an sich bekannte Weise isoliert werden, z.B. durch Extraktion, Destillation, Kristallisation oder Chromatographie. Eine solche Isolierung ist jedoch im allgemeinen nicht erforderlich. Die Verfahrensschritte 1 und 2 sowie 3 und 4 können somit als Eintopfverfahren durchgeführt werden, was einen weiteren Vorteil des erfindungsgemässen Verfahrens darstellt.

Die Zwischenprodukte der Formeln III und IV und die Salze von Verbindungen der Formel IV mit anorganischen oder organischen Säuren (R=H) sind neu und ebenfalls Gegenstand vorliegender Erfindung. Dabei stellt R bevorzugt H, –CHO, –COR' oder –COOR' dar und R' ist Methyl oder Äthyl.

Die Verbindung der Formel I stellt – wie eingangs erwähnt – ein bekanntes wertvolles Fungizid dar (vgl. DE-OS 2735872).

*Beispiel 1:*

*1-[2-(2,4-Dichlorphenyl)pentyliden]-2-acetylhydrazin*

Eine Lösung von 228 g (1 Mol) 2-(2,4-Dichlorphenyl)valeronitril, 74 g (1 Mol) Acetylhydrazid und 60 g Essigsäure in 2,3 Liter 95%igem wässerigem Äthanol wird mit 100 g Raney-Nickel versetzt und bei Raumtemperatur unter Wasserstoffnormaldruck während 8 Stunden hydriert. Filtration und Kristallisation ergeben 194,8 g (68% d.Th.) 1-[2-(2,4-Dichlorphenyl)pentyliden]-2-acetylhydrazin, das nach dem Umkristallisieren aus Methanol einen Smp. von 145-147° C und die folgenden analytischen Daten aufweist:

IR-Spektrum ($CHCl_3$) im $cm^{-1}$: 1672 (CO). $^1H$-NMR-Spektrum (60 MHz, $CDCl_3$) im ppm: 10,2 (bs, 1H, HN); 7,4-6,7 (m, 4H, 3H-Ar, $-C\underline{H}=N-$); 4,2-3,8 (m, 1H, HC(2)); 2,11 (s, 3H, $H_3C-CO-$); 2,2-0,7 (m, 7H, $H_3C-H_2C-H_2C-$). Massenspektrum: 289/287 ($M^+$+1).

Elementaranalyse für $C_{13}H_{16}Cl_2N_2O$ (Molgewicht 287,18):

Berechnet:
C 54,37% H 5,62% N 9,76% Cl 24,69%
Gefunden:
C 54,0 % H 5,7 % N 9,9 % Cl 24,7 %

*Beispiel 2:*

*1-[2-(2,4-Dichlorphenyl)pentyliden]-2-methoxycarbonylhydrazin*

Eine Lösung von 22,8 g (0,1 Mol) 2-(2,4-Dichlorphenyl)valeronitril, 9,0 g (0,1 Mol) Hydrazincarbonsäuremethylester und 6,0 g (0,1 Mol) Essigsäure in 200 ml 95%igem wässerigem Äthanol wird mit 11,0 g Raney-Nickel versetzt und bei Raumtemperatur unter Wasserstoffnormaldruck während 6,5 Stunden hydriert. Filtration und Kristallisation ergeben 23,3 g (77% d.Th.) des obigen Produkts, das nach dem Umkristallisieren aus Methanol einen Smp. von 165-166° C und die folgenden analytischen Daten aufweist:

IR-Spektrum ($CHCl_3$) in $cm^{-1}$: 1750, 1718. $^1H$-NMR-Spektrum (250 MHz, $CDCl_3$): 8,0 (bs, 1H, HN); 7,41 (d, J=2, 1H, H–Ar); 7,28 (s, 1H, HC=N–); 7,23 ($d_{AB}$xd, $J_{AB}$=8, J=2, 1H, H–Ar); 7,28 (s, 1H, HC=N–); 7,23 ($d_{AB}$xd, $J_{AB}$=8, J=2, 1H, H–Ar); 7,16 ($d_{AB}$, $J_{AB}$=8, 1H, H–Ar); 4,11 (q, J=7, 1H, HC(2)); 3,80 (s, 3H, $H_3CO$–); 2,02-1,68 (m, 2H, $H_2C$ (3)); 1,40-1,10 (m, 2H, $H_2C$ (4)); 0,88 (t, J=7, 3H, $H_3C$). Massenspektrum: 304/302 ($M^+$).

Elementaranalyse für $C_{13}H_{16}Cl_2N_2O_2$ (Molgewicht 303,19):

Berechnet:
C 51,50% H 5,32% N 9,24% O 10,56% Cl 23,39%

Gefunden:
C 51,7 % H 5,4 % N 9,3 % O 10,6 % Cl 23,5 %

*Beispiel 3:*

*1-[2-(2,4-Dichlorphenyl)pentyliden]-2-äthoxycarbonylhydrazin*

22,8 g (0,1 Mol) 2-(2,4-Dichlorphenyl)valeronitril, 10,4 g (0,1 Mol) Hydrazincarbonsäureäthylester und 6,0 g (0,1 Mol) Essigsäure werden in 100 ml 95%igem wässerigem Methanol mit 11,0 g Raney-Nickel bei Raumtemperatur unter Wasserstoffnormaldruck während 4,5 Stunden hydriert. Filtration und Kristallisation liefern 22,1 g (70% d.Th.) des obigen Produkts; Smp. 127-129° C.

$^1H$-NMR-Spektrum (60 MHz, $CDCl_3$) in ppm: 7,93 (bs, 1H, HN); 7,4-6,9 (m, 4H, 3 H–Ar, –C<u>H</u>=N–); 4,17 (q, J=7, 2H, –$H_2C$–O); 4,2-3,8 (m, 1H, –C<u>H</u>=N–); 2,2-0,6 (m, 10H).

Elementaranalyse für $C_{14}H_{18}Cl_2N_2O_2$ (Molgewicht 317,20):

Berechnet:
C 53,01% H 5,72% N 8,83% Cl 22,35%

Gefunden:
C 53,3 % H 5,7 % N 8,9 % Cl 22,4 %

*Beispiel 4:*

*1-[2-(2,4-Dichlorphenyl)pentyliden]-2-formylhydrazin*

22,8 g (0,1 Mol) 2-(2,4-Dichlorphenyl)valeronitril, 6,6 g (0,11 Mol) Formylhydrazid und 6,0 g (0,1 Mol) Essigsäure werden in 100 ml Isopropanol mit 11 g Raney-Nickel bei Raumtemperatur unter Wasserstoffnormaldruck während 24 Stunden hydriert. Durch Filtration des Reaktionsgemisches und Verteilung des Eindampfrückstandes zwischen Diäthyläther und Wasser erhält man nach Säulenchromatographie 11,4 g (42% d.Th.) des obigen Produkts.

IR-Spektrum ($CHCl_3$) in $cm^{-1}$: 1700 (CO).

$^1H$-NMR-Spektrum (250 MHz, $CDCl_3$) in ppm (J in Hz): 9,7-9,5 (b, 1H, HN); 8,67, 8,63 (2s, 1H, <u>H</u>CO); 7,41 (d, J=2, 1H, H–Ar); 7,26 (s, 1H, HC=N–); 7,24 ($d_{AB}$xd, $J_{AB}$=7, J=2, 1H, H–Ar); 7,15 ($d_{AB}$, $J_{AB}$=7, 1H, H–Ar); 4,08 (m, 1H, HC (2)); 2,05-1,65 (m, 2H, $H_2C$ (3)); 1,45-1,1 (m, 2H, $H_2C$ (4)); 0,90 (t, J=7, 3H, $H_3C$). Massenspektrum: 274/272 ($M^+$).

*Beispiel 5:*

Eine Lösung von 11,4 g (0,05 Mol) 2-(2,4-Dichlorphenyl)valeronitril, 3,7 g (0,05 Mol) Acetylhydrazid und 3,0 g (0,05 Mol) Essigsäure werden in 140 ml Methanol mit 2,0 g eines Rhodium/Kohle-Katalysators (5 Gew.-% Rh) versetzt und bei Raumtemperatur unter Wasserstoffnormaldruck während 8 Stunden hydriert. Man erhält das 1-[2-2,4-Dichlorphenyl)pentyliden]-2-acetylhydrazin in einer Ausbeute von 49% d.Th. (gaschromatographisch bestimmt).

*Beispiel 6:*

*1-[2-(2,4-Dichlorphenyl)pentyl]-2-acetylhydrazin*

a) Eine Lösung von 14,3 g (0,05 Mol) 1-[2-(2,4-Dichlorphenyl)pentyliden]-2-acetylhydrazin in 150 ml Isopropanol wird mit 2,5 g Raney-Nickel versetzt und bei 80° C unter 100 bar Wasserstoffdruck während 16 Stunden hydriert. Durch Filtration und Chromatographie des Eindampfrückstandes erhält man 10,9 g (76% d.Th.) des obigen Produkts.

$^1H$-NMR-Spektrum (60 MHz, $CDCl_3$) in ppm; in Gegenwart einer Spur Trifluoressigsäure: 8,32 (bs, 2H, 2HN); 7,4-7,0 (m, 3H, 3 H–Ar); 3,7-2,9 (m, 3H, $H_2C$ (1), HC (2)); 1,90 (s, 3H, $H_3C$); 2,0-0,6 (m, 7H).

b) Eine Lösung von 49,0 g (0,17 Mol) [1-(2,4-Dichlorphenyl)pentyliden]-2-acetylhydrazin in 170 ml abs. Äthanol wird mit 1,0 g eines Platin/Kohle-Katalysators (5 Gew.-% Pt) versetzt und bei 50° C unter 50 bar Wasserstoffdruck während 5 Stunden hydriert. Nach Abfiltrieren des Katalysators und Abdampfen des Äthanols erhält man 49,0 g eines öligen Rückstandes, der nach gaschromatographischer Bestimmung an 95 Gew.-% aus 1-[2-(2,4-Dichlorphenyl)pentyl]-2-acrylhydrazin besteht. Ausbeute: 95% d.Th.

*Beispiel 7*

*1-[2-(2,4-Dichlorphenyl)pentyl]-2-methoxycarbonylhydrazin*

Eine Lösung von 12,0 g (0,04 Mol) 1-[2-(2,4-Dichlorphenyl)pentyliden]-2-methoxycarbonylhydrazin in 80 ml Essigsäure wird mit 1,2 g eines Platin/Kohle-Katalysators (5 Gew.-% Pt) versetzt und bei 40° C unter 50 bar Wasserstoffdruck während 2 Stunden hydriert. Filtration des Reaktionsgemisches und Säulenchromatographie des Eindampfrückstandes liefern 9,4 g (78% d.Th.) des obigen Produkts.

$^1H$-NMR-Spektrum (250 MHz, $CDCl_3$) in ppm; in Gegenwart von $D_2O$: 7,39 (d, J=2, 1H, H–Ar); 7,27 (s, 1H, HC=N–); 7,26 ($d_{AB}$xd, $J_{AB}$=8, J=2, 1H, H–Ar); 7,21 ($d_{AB}$, $J_{AB}$=8, 1H, H–Ar); 4,80 (bs, DHO); 3,71 (s, 3H. $H_3CO$–); 3,50-3,36 (m, 1H, HC (2)); 3,20-2,95 (m, 2H, $H_2C$ (1)); 1,55-1,44 (m, 2H, $H_2C$ (3)); 1,35-1,05 (m, 2H, $H_2C$ (4)); 0,85 (t, J=7, 3H, $H_3C$).

*Beispiele 8-11*

Analog Beispiel 6 wird 1-[2-(Dichlorphenyl)-pentyliden]-2-acetylhydrazin unter den in der folgenden Tabelle angegebenen Reaktionsbedingungen zum 1-[2-(2,4-Dichlorphenyl)pentyl]-2-acetylhydrazin hydriert.

*Tabelle*

| Beispiel Nr. | Katalysator | $H_2$-Druck (bar) | Temperatur (°C) | Lösungsmittel** | Ausbeute (% d.Th.)* |
|---|---|---|---|---|---|
| 8 | Pt/C ( 5 Gew.-% Pt) | 50 | 100 | Methanol/Essigsäureäthylester | 61 |
| 9 | Ra–Ni | 50 | 100 | Methanol/Essigsäureäthylester | 89 |
| 10 | Ru/C (10 Gew.-% Ru) | 50 | 80 | Methanol/Essigsäureäthylester | 27 |
| 11 | Rh/C ( 5 Gew.-% Rh) | 50 | 100 | Methanol/Essigsäureäthylester | 37 |

* Gaschromatographisch bestimmt
** Im Volumenverhältnis von 1:1

*Beispiel 12:*

*1-[2-(2,4-Dichlorphenyl)pentyl]-1H-1,2,4-triazol*

5,2 g (0,018 Mol) 1-[2-(2,4-Dichlorphenyl)-pentyl]-2-acetyl-hydrazin werden in einem Gemisch von 10 ml Äthanol, 10 ml Wasser und 10 ml konzentrierter Salzsäure während 1 Stunde auf 90° C erhitzt. Anschliessend wird das erkaltete Reaktionsgemisch zwischen Diäthyläther und 2N Natronlauge verteilt, und die Ätherphase wird getrocknet und eingeengt. Der Eindampfrückstand wird in 50 ml Formamid langsam auf 180° C erhitzt und 2 Stunden bei dieser Temperatur gehalten. Anschliessend wird das abgekühlte Reaktionsgemisch zwischen Diäthyläther und Wasser verteilt. Durch Chromatographieren des Eindampfrückstandes erhält man 2,8 g (55% d.Th.) des obigen Produkts.

$^1$H-NMR-Spektrum (60 MHz, $CDCl_3$): identisch mit dem NMR-Spektrum von 1-[2-(2,4-Dichlorphenyl)pentyl]-1H-1,2,4-triazol, das durch Substitution des Methansulfonats von 2-(2,4-Dichlorphenyl)pentan-1-ol mit dem Natriumsalz von 1,2,4-Triazol hergestellt wurde; in ppm: 7,80 (s, 1H, H-Triazol); 7,67 (s, 1H, H-Triazol); 7,4-6,9 (m, 3H, $H_3Ar$); 4,37, 4,27 (2s, 2H, $H_2C$ (1)); 4,1-3,5 (m, 1H, HC (2)); 2,0-0,6 (m, 7H).

*Beispiel 13:*

5,1 g (0,017 Mol) 1-[2-(2,4-Dichlorphenyl)-pentyl]-2-acetylhydrazin werden in 10 ml Äthanol und 10 ml konzentrierter Salzsäure über Nacht bei 50° C gerührt. Dann wird das Reaktionsgemish zwischen Diäthyläther und 2N Natronlauge verteilt und der Eindampfrückstand wird in 50 ml absolutem Äthanol mit 5,75 g (0,035 Mol) [3-(Dimethylamino)-2-aza-prop-2-en-1-yliden]dimethylammoniumchlorid während 3,5 Stunden unter Rückfluss erhitzt. Dann wird die Reaktionslösung eingeengt, mit 50 ml Formamid versetzt und während 1,5 Stunden auf 170° C erhitzt. Durch Extraktion des mit Wasser versetzten Reaktionsgemisches mit Diäthyläther und Chromatographieren des Eindampfrückstandes erhält man 4,2 g (84% d.Th.) 1-[2-(2,4-Dichlorphenyl)pentyl-1H-1,2,4-triazol.

*Beispiel 14:*

*1-[2-(2,4-Dichlorphenyl)pentyl]-1,2-diformylhydrazin*

5,0 g (0,017 Mol) 1-[2-(2,4-Dichlorphenyl)-pentyl]-2-acetylhydrazin werden in 20 ml 85%iger wässeriger Ameisensäure während 18 Stunden auf 100° C erhitzt. Durch Säulenchromatographie des Eindampfrückstandes erhält man reines 1-[2-(2,4-Dichlorphenyl)pentyl]-1,2-diformylhydrazin.

IR-Spektrum ($CHCl_3$) in $cm^{-1}$: 1720, 1685 (CO). $^1$H-NMR-Spektrum (100 MHz, $CDCl_3$): $\delta$ in ppm; in Gegenwart von $D_2O$: 8,14, 8,13, 8,08, 7,93, 7,90, 7,73 (6s, 2H, 2C<u>H</u>O); 7,5-6,9 (m, 3H, 3H–Ar); 4,66 (s, 1H, HDO); 4,0-3,2 (m, 3H, $H_2C$ (1), HC (2)); 1,9-1,45 (m, 2H, $H_2C$ (3)); 1,45-1,0 (m, 2H, $H_2C$ (4)); 1,0-0,7 (m, 3H, $H_3C$ (5)).

*Beispiel 15:*

*1-[2-(2,4-Dichlorphenyl)pentyl]-1H-1,2,4-triazol*

6,2 g des gemäss Beispiel 14 erhaltenen 1-[2-(2,4-Dichlorphenyl)pentyl]-1,2-diformylhydrazins werden ohne Reinigung in 50 ml Formamid während 6 Stunden auf 170° C erhitzt. Chromatographie des erhaltenen Produkts liefert 1-[2-(2,4-Dichlorphenyl)pentyl]-1H-1,2,4-triazol, dessen $^1$H-NMR-Spektrum mit dem in Beispiel 12 beschriebenen $^1$H-NMR-Spektrum derselben Verbindung übereinstimmt.

*Beispiel 16:*

*1-[2-(2,4-Dichlorphenyl)pentyl]-1H-1,2,4-triazol*

57,8 g (0,2 Mol) 1-[2-(2,4-Dichlorphenyl)pentyl]-2-acetylhydrazin werden mit 216,5 g Ameisensäure (85%ig; 4,0 Mol) währen 20 Stunden am Rückfluss gekocht. Anschliessend werden flüchtige Anteile (Ameisensäure, Essigsäure und Wasser) bei 95°C/100 mbar abdestilliert. Der Rückstand

wird mit 38,5 g (0,61 Mol) Ammoniumformiat und 220 g (4,9 Mol) Formamid versetzt und 8 Stunden auf 160° C erhitzt.

Das auf 20° C abgekühlte Reaktionsgemisch wird mit Toluol versetzt. Nach Abtrennung der unteren, aus Formamid und Wasser bestehenden Schicht wird die toluolische Schicht mit 100 ml Wasser gewaschen und anschliessend eingedampft.

Man erhält 52,9 g eines langsam kristallisierenden Öls, das nach gaschromatographischer Analyse zu 90,1 Gew.-% aus 1-[2-(2,4-Dichlorphenyl)-pentyl]-1H-1,2,4-triazol besteht. Dies entspricht einer Ausbeute von 83,9% d.Th. bezogen auf eingesetztes 1-[2-(2,4-Dichlorphenyl)pentyl]-2-acetylhydrazin.

## Patentansprüche

1. Verfahren zur Herstellung des 1-[2-(2,4-Dichlorphenyl)pentyl]-1H-1,2,4-triazols der Formel I

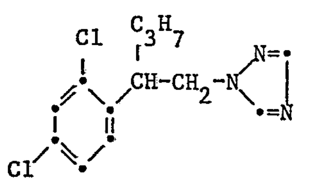

(I)

dadurch gekennzeichnet, dass man 2-(2,4-Dichlorphenyl)valeronitril in Gegenwart von Wasserstoff, einer Säure und eines Hydrierungskatalysators mit einer Verbindung der Formel II

$$H_2N-NH-R \qquad (II)$$

zu einer Verbindung der Formel III

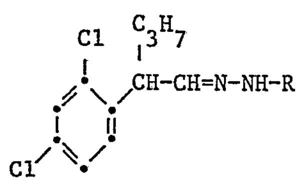

(III)

umsetzt, die Verbindung der Formel III katalytisch zu einer Verbindung der Formel IV

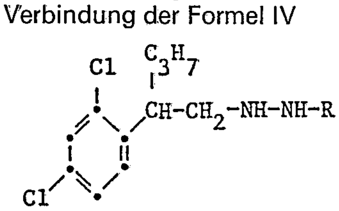

(IV)

hydriert, anschliessend entweder a) Verbindungen der Formel IV mit R ungleich Wasserstoff hydrolysiert und die Verbindungen der Formel IV mit R=H oder Salze davon mit anorganischen oder organischen Säuren mit Formamid und/oder [3-(Dimethylamino)-2-azaprop-2-en-1-yliden]-dimethylammoniumchlorid in die Verbindung der Formel I überführt, oder b) Verbindungen der Formel IV mit R=−COR′ mit wässeriger Ameisensäure in die N,N′-Bisformylderivate überführt und diese mit Formamid, gegebenenfalls in Gegenwart von $NH_3$ oder einer $NH_3$ liefernden Substanz zur Verbindung der Formel I umsetzt, wobei R Wasserstoff, −CHO, −COR′, −COOR′ oder $-CONH_2$ und R′ $C_{1-4}$-Alkyl, Benzyl oder Phenyl bedeuten.

2. Verfahren nach Anspruch 1, dadurch gekennzeichnet, dass man eine Verbindung der Formel II verwendet, worin R −CHO, −COR′ oder −COOR′ und R′ Methyl oder Äthyl darstellen.

3. Verfahren nach Anspruch 1, dadurch gekennzeichnet, dass man eine Verbindung der Formel II verwendet, worin R −COR″ und R″ Wasserstoff, Methyl oder Äthyl darstellen.

4. Verfahren nach Anspruch 1, dadurch gekennzeichnet, dass man die Umsetzung des 2-(2,4-Dichlorphenyl)valeronitrils mit dem Hydrazin der Formel II in organischem oder wässerig-organischem Medium vornimmt.

5. Verfahren nach Anspruch 1, dadurch gekennzeichnet, dass man die Umsetzung des 2-(2,4-Dichlorphenyl)valeronitrils mit dem Hydrazin der Formel II in einem $C_{1-4}$-Alkanol oder Gemischen davon mit Wasser vornimmt.

6. Verfahren nach Anspruch 1, dadurch gekennzeichnet, dass man für die Umsetzung des 2-(2,4-Dichlorphenyl)valeronitrils mit dem Hydrazin der Formel II als Hydrierungskatalysatoren Raney-Nickel oder Rhodium auf Trägern verwendet.

7. Verfahren nach Anspruch 1, dadurch gekennzeichnet, dass man die Umsetzung des 2-(2,4-Dichlorphenyl)valeronitrils mit dem Hydrazin der Formel II in Gegenwart einer aliphatischen Monocarbonsäure mit 1-4 C-Atomen vornimmt.

8. Verfahren nach Anspruch 1, dadurch gekennzeichnet, dass man die Hydrierung der Hydrazone der Formel III zu den Hydrazinen der Formel IV in Gegenwart eines organischen Lösungsmittels, besonders Essigsäure, ferner Methanol, Äthanol, Isopropanol, sek.-Butanol oder tert.-Butanol oder Gemischen dieser Alkohole mit Essigsäureäthylester durchführt.

9. Verfahren nach Anspruch 1, dadurch gekennzeichnet, dass man als Katalysatoren für die Hydrierung der Hydrazone der Formel III zu den Hydrazinen der Formel IV Raney-Nickel, Rhodium- oder Platinkatalysatoren verwendet.

10. Verfahren nach Anspruch 1, dadurch gekennzeichnet, dass man 2-(2,4-Dichlorphenyl)-valeronitril in Gegenwart von Essigsäure und eines Hydrierungskatalysators auf einem Acylhydrazin der Formel IIa

$$H_2N-NH-COR'' \qquad (IIa)$$

zu einem 1-[2-(2,4-Dichlorphenyl)pentyliden]-2-acylhydrazin der Formel IIIa

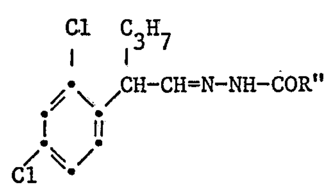

(IIIa)

umsetzt, dieses zum 1-[2-(2,4-Dichlorphenyl)-pentyl]-2-acylhydrazin der Formel IIIa

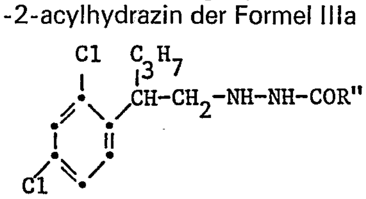

(IVa)

hydriert und dieses durch Umsetzung mit Ameisensäure zunächst in 1-[2-(2,4-Dichlorphenyl)-pentyl]-1,2-diformylhydrazin und dieses durch weitere Umsetzung mit Formamid bei 120-180° C in 1-[2-(2,4-Dichlorphenyl)pentyl]-1H-1,2,4-triazol überführt, wobei R″ in Formeln IIa, IIIa und IVa Wasserstoff, Methyl oder Äthyl bedeutet.

11. Verbindungen der Formeln III und IV

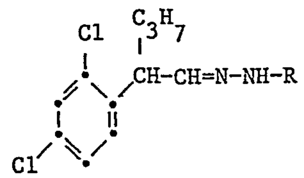

(III)

und

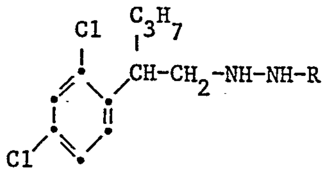

(IV)

worin R Wasserstoff, $-CHO$, $-COR'$, $-COOR'$ oder $-CONH_2$ und R′ $C_{1-4}$-Alkyl, Benzyl oder Phenyl bedeuten und Salze von Verbindungen der Formel IV mit R=H mit anorganischen oder organischen Säuren.

12. Verbindungen der Formeln III und IV nach Anspruch 9, worin R $-H$, $-CHO$, $-COR'$ oder $-COOR'$ und R′ Methyl oder Äthyl darstellen.

## Claims

1. A process for the preparation of 1-[2-(2,4-dichlorophenyl)pentyl]-1H-1,2,4-triazole of the formula I

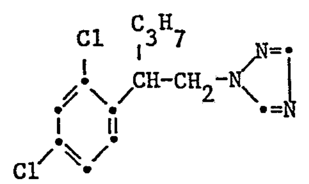

(I)

which process comprises reacting 2-(2,4-dichlorophenyl)valeronitrile, in the presence of hydrogen, an acid and a hydrogenation catalyst, with a compound of the formula II

$$H_2N-NH-R \qquad (II)$$

to give a compound of the formula III

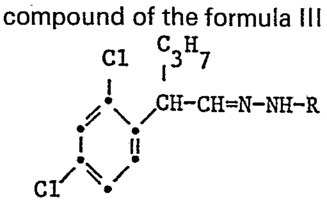

(III)

hydrogenating the compound of the formula III catalytically to give a compound of the formula IV

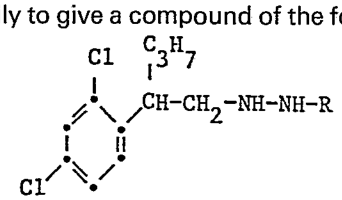

(IV)

and subsequently either a) hydrolysing a compound of the formula IV wherein R is not hydrogen, and converting the compound of the formula IV wherein R=H, or a salt thereof with an inorganic or organic acid, with formamide, and/or with [3-(dimethylamino)-2-azaprop-2-en-1-ylidene]-dimethylammonium chloride, into the compound of the formula I, or b) converting a compound of the formula IV wherein R is $-COR'$, with aqueous formic acid, into an N,N′-bisformyl derivative, and reacting this with formamide, optionally in the presence of $NH_3$ or of an $NH_3$ donor, into a compound of the formula I; where R is hydrogen, $-CHO$, $-COR'$, $-COOR'$ or $-CONH_2$, and R′ is $C_1$-$C_4$alkyl, benzyl or phenyl.

2. A process according to claim 1, which comprises the use of a compound of the formula II, wherein R is $-CHO$, $-COR'$ or $-COOR'$, and R′ is methyl or ethyl.

3. A process according to claim 1, which comprises the use of a compound of the formula II, wherein R is $-COR''$, and R″ is hydrogen, methyl or ethyl.

4. A process according to claim 1, wherein the reaction of 2-(2,4-dichlorophenyl)valeronitrile with the hydrazine of the formula II is performed in an organic or aqueous-organic medium.

5. A process according to claim 1, wherein the reaction of 2-(2,4-dichlorophenyl)valeronitrile with the hydrazine of the formula II is performed in a $C_1$-$C_4$alkanol, or in a mixture thereof with water.

6. A process according to claim 1, wherein the hydrogenation catalyst for the reaction of 2-(2,4-dichlorophenyl)valeronitrile with the hydrazine of the formula II is Raney nickel or rhodium on a carrier.

7. A process according to claim 1, wherein the reaction of 2-(2,4-dichlorophenyl)valeronitrile with the hydrazine of the formula II is performed in the presence of an aliphatic monocarboxylic acid having 1 to 4 carbon atoms.

8. A process according to claim 1, wherein the hydrogenation of a hydrazone of the formula III to give a hydrazine of the formula IV is performed in the presence of an organic solvent, in particular acetic acid, and also methanol, ethanol, isopropanol, sec-butanol or tert-butanol, or of a mixture of this alcohol with ethyl acetate.

9. A process according to claim 1, wherein the catalyst for the hydrogenation of the hydrazone of the formula III to the hydrazine of the formula IV is a Raney nickel, rhodium or platinum catalyst.

10. A process according to claim 1, which comprises reacting 2-(2,4-dichlorophenyl)valeronitrile, in the presence of acetic acid and a hydrogenation catalyst, with an acylhydrazine of the formula IIa

$$H_2N-NH-COR'' \qquad (IIa)$$

to give a 1-[2-(2,4-dichlorophenyl)pentylidene]-2-acylhydrazine of the formula IIIa

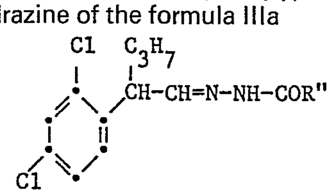

(IIIa)

hydrogenating this to 1-[2-(2,4-dichlorophenyl)-pentyl]-2-acylhydrazine of the formula IVa

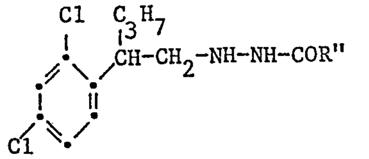
(IVa)

and converting this, by reaction with formic acid, first into 1-[2-(2,4-dichlorophenyl)pentyl]-1,2-diformylhydrazine, and this, by further reaction with formamide at 120-180°C, into 1-[2-(2,4-dichlorophenyl)pentyl]-1H-1,2,4-triazole; the symbol R'' in the formulae IIa, IIIa and IVa being hydrogen, methyl or ethyl.

11. Compounds of the formulae III and IV

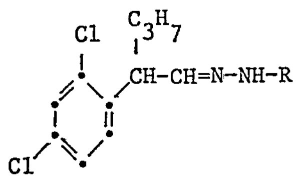
(III)

and

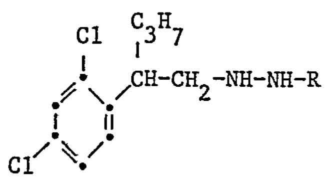
(IV)

wherein R is hydrogen, −CHO, −COR', −COOR' or $-CONH_2$, and R' is $C_1$-$C_4$alkyl, benzyl or phenyl, and salts of compounds of the formula IV, wherein R=H, with inorganic or organic acids.

12. Compounds of the formulae III and IV according to claim 9, wherein R is −H, −CHO, −COR' or −COOR', and R' is methyl or ethyl.

**Revendications**

1. Procédé de préparation du 1-[2-(2,4-dichlorophényl)pentyl]-1H-1,2,4-triazole de formule I

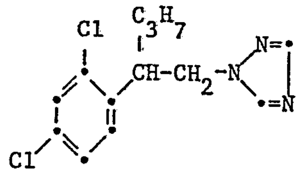
(I)

caractérisé en ce que l'on fait réagir le 2-(2,4-dichlorophényl)valéronitrile en présence d'hydrogène, d'un acide et d'un catalyseur d'hydrogénation, avec un composé de formule II

$$H_2N-NH-R \quad (II)$$

ce qui donne un composé de formule III

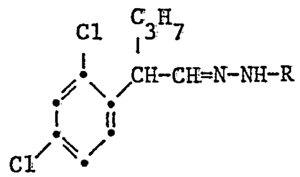
(III)

qu'on convertit par hydrogénation catalytique en un composé de formule IV

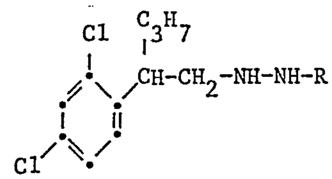
(IV)

après quoi, ou bien a) on hydrolyse les composés de formule IV dans laquelle R a une signification autre que l'hydrogène et on convertit les composés de formule IV dans laquelle R=H ou les sels de ces composés et d'acides minéraux ou organiques, à l'aide du formamide et/ou du chlorure de [3-(diméthylamino)-2-azapropa-2-ène-1-ylidène]-diméthylammonium en le composé de formule I, ou bien b) on convertit les composés de formule IV dans laquelle R=−COR', à l'aide de l'acide formique aqueux, en les dérivés N,N'-bis-formylés qu'on fait réagir avec le formamide, éventuellement en présence de $NH_3$ ou d'une substance apportant $NH_3$, cette réaction donnant le composé de formule I dans laquelle R représente l'hydrogène, −CHO, −COR', −COOR' ou $-CONH_2$, R' représentant un groupe alkyle en $C_1$-$C_4$, benzyle ou phényle.

2. Procédé selon la revendication 1, caractérisé en ce que l'on utilise un composé de formule II dans laquelle R représente −CHO, −COR', −COOR' et R' représente un groupe méthyle ou éthyle.

3. Procédé selon la revendication 1, caractérisé en ce que l'on utilise un composé de formule II dans laquelle R représente −COR'' et R'' représente l'hydrogène, un groupe méthyle ou éthyle.

4. Procédé selon la revendication 1, caractérisé en ce que la réaction du 2-(2,4-dichlorophényl)-valéronitrile avec l'hydrazine de formule II est effectuée en milieu organique ou hydro-organique.

5. Procédé selon la revendication 1, caractérisé en ce que la réaction du 2-(2,4-dichlorophényl)-valéronitrile avec l'hydrazine de formule II est effectuée dans un alcanol en $C_1$-$C_4$ ou un mélange d'un tel alcanol et d'eau.

6. Procédé selon la revendication 1, caractérisé en ce que, pour la réaction du 2-(2,4-dichlorophényl)valéronitrile avec l'hydrazine de formule II, on utilise en tant que catalyseur d'hydrogénation le nickel de Raney ou le rhodium sur support.

7. Procédé selon la revendication 1, caractérisé en ce que la réaction du 2-(2,4-dichlorophényl)-valéronitrile avec l'hydrazine de formule II est effectuée en présence d'un acide monocarboxylique aliphatique en $C_1$-$C_4$.

8. Procédé selon la revendication 1, caractérisé en ce que l'hydrogénation des hydrazones de formule III en les hydrazines de formule IV est effectuée en présence d'un solvant organique, plus spécialement l'acide acétique ou encore le méthanol, l'éthanol, l'isopropanol, le sec-butanol ou le tert-butanol ou des mélanges de ces alcools avec l'acétate d'éthyle.

9. Procédé selon la revendication 1, caractérisé en ce que, pour l'hydrogénation des hydrazones de

formule III en les hydrazines de formule IV on utilise en tant que catalyseurs du nickel de Raney, des catalyseurs au rhodium ou au platine.

10. Procédé selon la revendication 1, caractérisé en ce que l'on fait réagir le 2-(2,4-dichlorophényl)valéronitrile en présence d'acide acétique et d'un catalyseur d'hydrogénation avec une acylhydrazine de formule IIa

$$H_2N-NH-COR'' \qquad (IIa)$$

la réaction donnant une 1-[2-(2,4-dichlorophényl)pentylidène]-2-acylhydrazine de formule IIIa

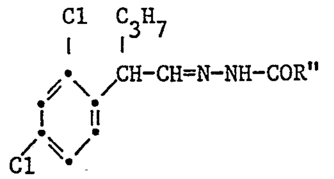
(III)a)

qu'on convertit par hydrogénation en la 1-[2-(2,4-dichlorophényl)pentyl]-2-acylhydrazine de formule IIIa

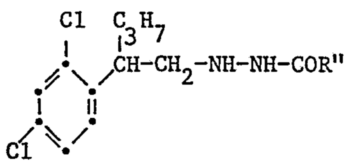
(IVa)

qu'on convertit d'abord, par réaction avec l'acide formique, en la 1-[2-(2,4-dichlorophényl)pentyl]-1,2-diformylhydrazine, laquelle est ensuite convertie par une nouvelle réaction avec le formamide à une température de 120 à 180° C, en le 1-[2-(2,4-dichlorophényl)pentyl]-1H-1,2,4-triazole, le symbole R'' représentant, dans les formules IIa, IIIa et IVa, l'hydrogène, un groupe méthyle ou éthyle.

11. Composés de formules III et IV

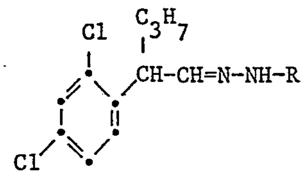
(III)

et

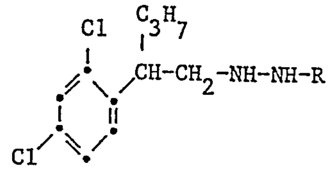
(IV)

dans lesquelles R représente l'hydrogène, $-CHO$, $-COR'$, $-COOR'$ ou $-CONH_2$ et R' représente un groupe alkyle en $C_1$-$C_4$, benzyle ou phényle, et les sels de composés de formule IV dans laquelle R=H et d'acides minéraux ou organique.

12. Composés de formules III et IV selon la revendication 9, dans lesquelles R représente $-H$, $-CHO$, $-COR'$ ou $-COOR'$ et R' représente un groupe méthyle ou éthyle.